# EUROPÄISCHE PATENTSCHRIFT

(11) **EP 1 240 157 B1**
(45) Veröffentlichungstag und Bekanntmachung des Hinweises auf die Patenterteilung: **11.02.2004**
(21) Anmeldenummer: 00983349.2
(22) Anmeldetag: 19.12.2000
(51) Int. Cl.: C07D 401/12, A61K 31/404, A61P 35/00

(54) **SUBSTITUIERTE N-BENZYL-INDOL-3-YL- GLYOXYLSÄURE-DERIVATE MIT ANTITUMORWIRKUNG**
SUBSTITUTED N-BENZYL-INDOL-3-YL GLYOXYLIC ACID DERIVATIVES HAVING AN ANTI-TUMORAL EFFECT
DERIVES SUBSTITUES D'ACIDE N-BENZYL-INDOL-3-YL-GLYOXALIQUE A ACTION ANTITUMORALE

(30) Priorität: 23.12.1999 DE 19962300
(43) Veröffentlichungstag der Anmeldung: 18.09.2002
(73) Patentinhaber: Zentaris GmbH, 60314 Frankfurt/Main (DE)
(72) Erfinder: GÜNTHER, Eckhard, 63477 Maintal (DE); EMIG, Peter, 63486 Bruchköbel (DE); REICHERT, Dietmar, 63863 Eschau (DE); LE BAUT, Guillaume, F-44230 Saint Sebastien (FR); NICKEL, Bernd, 64367 Mühltal (DE); BACHER, Gerald, 82110 Germering (DE)
(86) Internationale Anmeldenummer: PCT/EP2000/012947
(87) Internationale Veröffentlichungsnummer: WO 2001/047913

(56) Entgegenhaltungen:
- WO-A-00/67802
- WO-A-01/22954
- WO-A-98/09946
- WO-A-99/43654
- WO-A-99/51224
- WO-A-99/55696
- POLYMEROPOULOS ET AL.: "A peptidic binding site model for PDE 4 inhibitors" QUANT. STRUCT. -ACT. RELAT., Bd. 18, Nr. 6, 1999, Seiten 543-7, XP001030741
- BACHER, GERALD ET AL: "D-24851, a novel synthetic microtubule inhibitor, exerts curative antitumoral activity in vivo, shows efficacy toward multidrug-resistant tumor cells, and lacks neurotoxicity" CANCER RES. (2001), 61(1), 392-399, XP002173885
- ANDRADE-GORDON P ET AL: "Design, synthesis, and biological characterization of a peptide-mimetic antagonist for a tethered-ligand receptor" PROCEEDINGS OF THE NATIONAL ACADEMY OF SCIENCES OF USA, NATIONAL ACADEMY OF SCIENCE. WASHINGTON, US, Bd. 96, Nr. 22, 26. Oktober 1999 (1999-10-26), Seiten 12257-12262, XP002157104 ISSN: 0027-8424

## Beschreibung

Indol-3-glyoxylamide finden als pharmakodynamische Verbindungen und als Synthesebausteine in der pharmazeutischen Chemie eine vielfältige Verwendung.

In der Patentanmeldung Neth. Appl. 6502481 sind Verbindungen beschrieben, die über ein antiinflammatorisches und antipyretisches Wirkprofil und analgetische Aktivität verfügen.

In der britischen Anmeldung GB-PS 1 028 812 finden Derivate der Indol-3-glyoxylsäure und deren Amide Erwähnung als analgetisch, antikonvulsiv und β-adrenergisch wirksame Verbindungen.

G. Domschke et al. (Ber. 94, 2353 (1961)) beschreibt 3-Indolyl-glyoxylamide, die pharmakologisch nicht charakterisiert sind.

E. Walton berichtet in J. Med. Chem 11, 1252 (1968) über Indolyl-3-glyoxylsäure-Derivate, die inhibitorisch auf die Glycerophosphat-Dehydrogenase und Lactat-Dehydrogenase wirken.

In der Europäischen Patentschrift EP 675 110 werden 1H-Indol-3-glyoxylsäureamide beschrieben, die als sPLA2-inhibitoren profiliert werden und bei Behandlung des septischen Schocks, bei Pankreatitis, bei der Behandlung allergischer Rhinitis und rheumatischer Arthritis zur Anwendung kommen.

Es wurde ferner in der deutschen Patentanmeldung mit dem Aktenzeichen 198 14 838.0 bereits vorgeschlagen, die Verbindungen gemäß DE-OS 196 36 150 A1 als Antitumormittel einzusetzen.

In der Patentschrift WO 99/51224 werden Derivate der Indolyl-3-glyoxylsäure veröffentlicht, die zur Behandlung von Tumorerkrankungen eingesetzt werden können. Es werden Verbindungen beschrieben, bei denen der Indol-Stickstoff mit Arylalkyl-Gruppen substituiert sein kann, bei denen der Arylbaustein jedoch keine Stickstoffsubstitution besitzt.

E. Polymeropoulos et al. berichtet in "A peptidic binding site model for PDE 4 inhibitors", Quant. Struct.-Act. Relat., Bd. 18, Nr. 6, 1999, Seiten 543-7, XP001030741 über ein neuartiges peptidisches Bindungsmodell von PDE-4-inhibitorisch aktiven 5-Methoxy- und 5-Hydroxy-indol-3-yl-glyoxylsäure-Derivaten.

Ziel der vorliegenden Erfindung ist es, neue Verbindungen aus der Indol-3-ylglyoxylsäure-Reihe der allgemeinen Formel 1 zur Verfügung zu stellen, die eine gute antitumoraktive Wirkung besitzen und zur Herstellung von Antitumormitteln eingesetzt werden können; 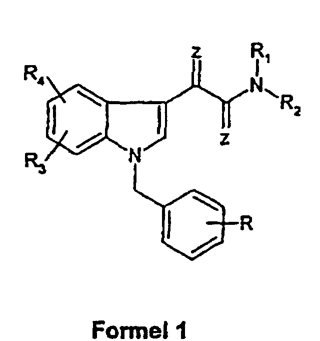 Wobei die Reste R, R₁, R₂, R₃, R₄ und Z die folgende Bedeutung haben
- R =: Nitro, Amino, Mono-bzw. Di(C₁-C₆)-alkylamino, Mono- bzw. Di(C₁-C₆)-cycloalkylamino, (C₁-C₆)-Acylamino, Phenyl (C₁-C₆)-alkylamino, Aroytamino, Heteroaroylamino, (C₁-C₆)-Alkylsulfonamido, Arylsulfonamido, Maleinimido, Succinimido, Phthalimido, Benzyloxycarbonylamino (Z-Amino), tert.-Butoxycarbonylamino (BOC-Amino). 9-Fluorenylmethoxycarbonylamino (Fmoc-Amino), Triphenylmethylamino (Tr-Amino), 2-(4'-Pyridyl)-ethoxycarbonylamino (Pyoc-Amino), Diphenylmethylsilyl-amino (DPMS-Amino), wobei die Reste für R wahlweise an den C-Atomen 2,3 und 4 des Phenylrings substituiert sein können,
- R: kann weiterhin für den Fall, daß R₁=Wasserstoff, die Methyl-oder Phenylmethylgruppe sowie den Benzyloxycarbonyl-Rest (Z-Rest), den tert.-Butoxycarbonyl-Rest (BOC-Rest) und die Acetylgruppe darstellt, die folgenden Reste bedeuten:
- NH-CH₂-COOH; -NH-CH(CH₃)-COOH;
(CH₃)₂CH-CH₂-CH₂-CH(NH)-COOH; H₃C-CH₂-CH(CH₃)-CH(NH)-COOH;
HOH₂C-CH(NH)-COOH; Phenyl-CH₂-CH(NH)-COOH; (4-Imidazoyl)-CH₂-CH(NH)-COOH;
HN=C(NH₂)-NH-(CH₂)₃-CH(NH)-COOH; H₂N-(CH₂)₄-CH(NH)-COOH;
H₂N-CO-CH₂-CH(NH)-COOH; HOOC-(CH₂)₂-CH(NH)-COOH
- R₁=: Wasserstoff, (C₁-C₆)-Alkyl, wobei die Alkylgruppe ein- oder mehrfach durch den Phenylring substituiert sein kann und dieser Phenylring seinerseits ein- oder mehrfach durch Halogen, (C₁-C₆)-Alkyl, (C₃-C₇)-Cycloalkyl, durch Carboxylgruppen, mit C₁-C₆-Alkanolen veresterte Carboxylgruppen, Trifluormethylgruppen, Hydroxylgruppen, Methoxygruppen, Ethoxygruppen, Benzyloxygruppen sowie durch eine im Phenylteil ein- oder mehrfach mit (C₁-C₆)- Alkylgruppen, Halogenatomen oder Trifluormethylgruppen substituierte Benzylgruppe substituiert sein kann,
- R₁: steht ferner für die Benzyloxycarbonyl-Gruppe (Z-Gruppe) und für den tertiär-Butoxycarbonylrest (Boc-Rest), weiterhin für die Acetylgruppe.
- R₂: kann den Phenylring, der ein- oder mehrfach mit (C₁-C₆)-Alkyl, (C₁-C₆)-Alkoxy, Cyano, Halogen, Trifluormethyl, Hydroxy, Benzyloxy, Nitro, Amino, (C₁-C₆)-Alkylamino, (C₁-C₆)-Alkoxycarbonyl-amino und mit der Carboxylgruppe bzw. mit der mit C₁-C₆- Alkanolen veresterten Carboxylgruppe substituiert ist, oder ein Pyridin-Gerüst der Formel 2 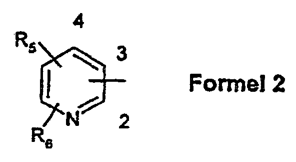 bedeuten und dessen N-Oxid, wobei das Pyridin-Gerüst wahlweise an den Ringkohlenstoff Atomen 2, 3 und 4 gebunden ist und mit den Substituenten R₅ und R₆ substituiert sein kann. Die Reste R₅ und R₆ können gleich oder verschieden sein und die Bedeutung (C₁-C₆)-Alkyl sowie die Bedeutung (C₃-C₇)-Cycloalkyl, (C₁-C₆)-Alkoxy, Nitro, Amino, Hydroxy, Halogen und Trifluormethyl besitzen und ferner den Ethoxycarbonylamino-Rest sowie die Gruppe Carboxyalkyloxy darsteilen, bei dem die Alkylgruppe über 1-4 C-Atome verfügen kann.
- R₂: kann ferner ein 2-bzw. 4-Pyrimidinyl-Heterocyclus sein, wobei der 2-Pyrimidinyl-Ring ein- oder mehrfach mit der Methylgruppe substituiert sein kann, weiterhin das mit (C₁-C₆)-Alkyl, Halogen, der Nitrogruppe, der Aminogruppe und dem (C₁-C₆)-Alkylamino-Rest substituierte 2-,3-,4-,5-,6-,7- und 8-Chinolylgerüst bedeuten, eine 2-, 3- und 4-Chinolylmethylgruppe darstellen, wobei die Ringkohlenstoffe des Pyridylmethyirestes der Chinolylgruppe und des Chinolylmethyl-Restes mit (C₁-C₆)-Alkyl, (C₁-C₆)-Alkoxy, Nitro, Amino und (C₁-C₆)-Alkoxycarbonylamino substituiert sein können.
- R₂: kann weiterhin für den Fall, daß R₁=Wasserstoff, die Methyl-oder Benzylgruppe sowie den Benzyloxycarbonyl-Rest (Z-Rest), den tert.-Butoxycarbonyl-Rest (BOC-Rest) und die Acetylgruppe darstellt, die folgenden Reste bedeuten:
- CH₂COOH;-CH(CH₃)-COOH;(CH₃)₂CH-(CH₂)₂-CH(COOH)-; H₃C-H₂C-CH(CH₃)-CH(COOH)-;
HO-H₂C-CH(COOH)-; Phenyl-CH₂-CH(COOH)-; (4-Imidazolyl)-CH₂-CH(COOH)-;
HN=C(NH₂)-NH-(CH₂)₃-CH(COOH)-; H₂N-(CH₂)₄-CH(COOH)-; H₂N-CO-CH₂-CH(COOH)-;
HOOC-(CH₂)₂-CH(COOH)-;
- R₂: kann weiterhin für den Fall, daß R₁ Wasserstoff, die Z-Gruppe, den BOC-Rest, die Acetyl- oder die Benzylgruppe bedeuten, der Säurerest einer natürlichen oder unnatürlichen Aminosäure sein, z.B. den α-Glycyl-, den α-Sarkosyl-, den α-Alanyl-, den α-Leucyl-, den α-iso-Leucyl-, den α-Seryl-, den α-Phenylalanyl-, den α-Histidyl-, den α-Prolyl-, den α-Arginyl-, den α-Lysyl-, den α-Asparagyl- und den α-Glutamyl-Rest darstellen, wobei die Aminogruppen der jeweiligen Aminosäuren ungeschützt vorliegen oder geschützt sein können. Als Schutzgruppe der Aminofunktion kommen der Carbobenzoxy-Rest (Z-Rest) und der tert.-Butoxycarbonyl-Rest (BOC-Rest) sowie die Acetylgruppe in Frage. Im Fall des für R₂ beanspruchten Asparagyl-und Glutamylrestes liegt die zweite, nicht gebundene Carboxylgruppe als freie Carboxylgruppe oder in Form einer mit C₁-C₆-Alkanolen veresterten Carboxylgruppe, z.B. als Methyl-, Ethyl- bzw. als tert.- Butylester vor. Weiterhin kann R₂ die Allylaminocarbonyl-2-methyl-prop-1-yl-Gruppe bedeuten. R₁ und R₂ können ferner zusammen mit dem Stickstoff-Atom, an das sie gebunden sind, einen Piperazinring der Formel 3 oder einen Homopiperazinring bilden, sofern R₂ eine Aminoalkylengruppe darstellt, bei dem 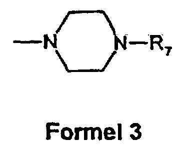 R₇ einen Alkylrest darstellt, einen Phenylring bedeutet, der ein- oder mehrfach mit (C₁-C₆)-Alkyl, (C₁-C₆)-Alkoxy, Halogen, der Nitrogruppe, der Aminofunktion und mit der (C₁-C₆)-Alkylaminogruppe substituiert sein kann. R₇ bedeutet ferner die Benzhydryl-Gruppe und die Bis-p-fluorbenzhydryl-Gruppe.
- R₃ und R₄: können gleich oder verschieden sein und Wasserstoff, (C₁-C₆)-Alkyl, (C₃-C₇)-Cycloalkyl, (C₁-C₆)-Alkanoyl, Halogen und Benzyloxy bedeuten. Weiterhin können R₃ und R₄ die Nitrogruppe, die Aminogruppe, die (C₁-C₄)-mono- oder dialkylsubstituierte Aminogruppe und die (C₁-C₆)-Alkoxy-carbonylamino-Funktion oder die (C₁-C₆)-Alkoxycarbonylamino-(C₁-C₆)-alkyl-Funktion bedeuten.
- Z: steht für O und S

Unter der Bezeichnung Alkyl-, Alkanol-, Alkoxy- oder Alkylaminogruppe sind für die Reste R, R₁, R₂, R₃, R₄, R₅, R₆, R₇ regelmäßig sowohl "geradkettige" als auch "verzweigte" Alkylgruppen zu verstehen, wobei "geradkettige Alkylgruppen" beispielsweise Reste wie Methyl, Ethyl, n-Propyl, n-Butyl, n-Pentyl, n-Hexyl bedeuten können und "verzweigte Alkylgruppen" beispielsweise Reste wie Isopropyl oder tert.-Butyl bezeichnen. Unter "Cycloalkyl" sind Reste wie beispielsweise Cyclopropyl, Cyclobutyl, Cyclopentyl, Cyclohexyl oder Cycloheptyl zu verstehen. Die Bezeichnung "Halogen" steht für Fluor, Chlor, Brom oder Jod. Die Bezeichnung "Alkoxygruppe" stellt Reste wie beispielsweise Methoxy, Ethoxy, Propoxy, Butoxy, Isopropoxy, Isobutoxy oder Pentoxy dar. Unter der Bezeichnung Acyl der Acylamino-Reste sind die Gruppen Formyl, Acetyl, Propionyl, Butyryl, Valeryl und Isovaleryl zu verstehen. Die Bezeichnung Aroyl der Aroylamino-Gruppen steht für Benzoyl, Naphthoyl, Toluoyl, Phthaloyl und die Gruppierung Heteroaroyl der Heteroaroylamino-Reste steht für Nicotinoyl, Isonicotinoyl, Thenoyl und Furoyl. Unter der Bezeichnung Aryl der Arylsulfonamido-Gruppe versteht man Phenyl, Tolyl und Naphthyl.
Die Verbindungen können auch als Säureadditionssalze eingesetzt werden, beispielsweise als Salze von Mineralsäuren, wie beispielsweise Salzsäure, Schwefelsäure, Phosphorsäure, Salze von organischen Säuren, wie beispielsweise Essigsäure, Milchsäure, Malonsäure, Maleinsäure, Fumarsäure, Gluconsäure, Glucuronsäure, Zitronensäure, Ascorbinsäure, Embonsäure, Methansulfonsäure, Trifluoressigsäure, Bernsteinsäure, 2-Hydroxy-ethansulfonsäure, Nicotinsäure und p-Toluol-sulfonsäure.
Sowohl die Verbindungen der Formel 1 als auch deren Salze sind biologisch aktiv.Die Verbindungen der Formel 1 können in freier Form oder als Salze mit physiologisch verträglichen Säuren verabreicht werden.
Die Applikation kann peroral, parenteral, intravenös, transdermal oder inhalativ vorgenommen werden.
Weiterhin betrifft die Erfindung pharmazeutische Zubereitungen mit einem Gehalt an mindestens einer der Verbindungen der Formel 1 oder deren Salze mit physiologisch verträglichen anorganischen oder organischen Säuren und gegebenenfalls pharmazeutisch verwendbaren Träger- und/oder Verdünnungs- bzw. Hilfsstoffen.
Als Applikationsformen eignen sich beispielsweise Tabletten, Dragees, Kapseln, Lösungen zur Infusion oder Ampullen, Suppositorien, Pflaster, inhalativ einsetzbare Pulverzubereitungen, Suspensionen, Cremes und Salben.

Die Verfahren zur Herstellung der erfindungsgemäßen Verbindungen werden in den folgenden Reaktionsschemata 1 u. 2 (Stufen 1-3) sowie in allgemeinen Vorschriften beschrieben. Alle Verbindungen lassen sich, wie beschrieben, oder analog herstellen:
Die Verbindungen der allgemeinen Formel 1 mit Z=O, R=NO₂ und NH₂ und R₂=Aryl, Aralkyl und Heteroaryl sind gemäß des folgenden Schemas 1 erhältlich:

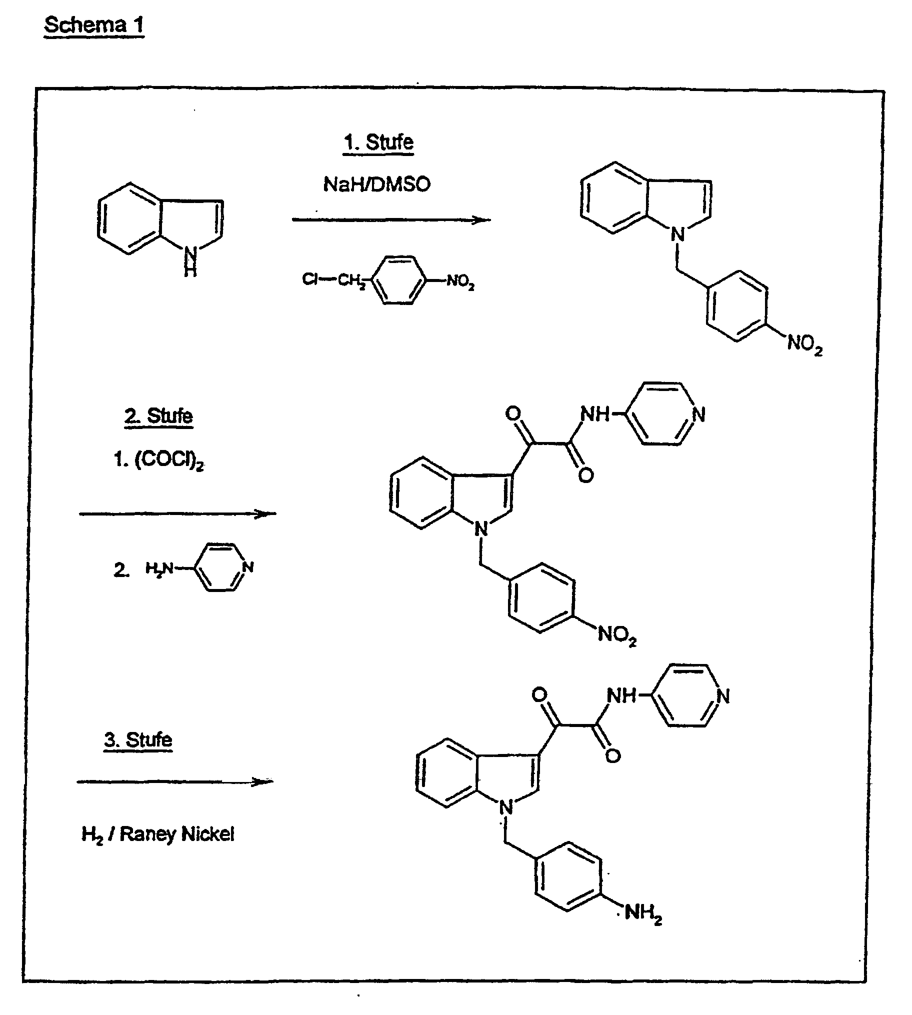

### 1.Stufe:

Das Indol-Derivat, das unsubstituiert oder an C-2 oder im Phenylgerüst einfach oder mehrfach substituiert sein kann, wird in einem protischen, dipolar aprotischen oder unpolaren organischen Lösungsmittel, wie beispielsweise Isopropanol, Tetrahydrofuran, Dimethylsulfoxid, Dimethylformamid, Dimethylacetamid, N-Methylpyrrolidon, Dioxan, Toluol oder Methylenchlorid gelöst und tropfenweise zu einer in einem Dreihalskolben unter N₂-Atmosphäre vorbereiteten molaren oder überschüssig eingesetzten Suspension einer Base, wie beispielsweise Natriumhydrid, pulverisiertes Kaliumhydroxid, Kalium-tert.butylat, Dimethylaminopyridin oder Natriumamid in einem geeigneten Lösungsmittel gegeben. Sodann gibt man beispielsweise das gewünschte Alkyl-, Aralkyl-bzw. Heteroaralkylhalogenid gegebenenfalls unter Zusatz eines Katalysators, wie z.B. Kupfer, zu und läßt einige Zeit, beispielsweise 30 Minuten bis 12 Stunden, nachreagieren und hält die Temperatur innerhalb eines Bereichs von 0°C bis 120°C, vorzugsweise zwischen 30°C bis 80°C, besonders zwischen 50°C und 65°C. Nach Beendigung der Reaktion wird die Reaktionsmischung in Wasser gegeben, die Lösung z.B. mit Diethylether, Dichlormethan, Chloroform, Methyl-tert.-butytether oder Tetrahydrofuran extrahiert und die jeweils erhaltene organische Phase mit wasserfreiem Natriumsulfat getrocknet. Man engt die organische Phase im vakuum ein, kristallisiert den verbleibenden Rückstand durch Anreiben oder reinigt den öligen Rückstand durch Umkristallisation, Destillation oder durch Säulen- bzw. Flash-Chromatographie an Kieselgel oder Aluminiumoxid. Als Laufmittel dient beispielsweise ein Gemisch aus Dichlormethan und Diethylether im Verhältnis 8:2 (Vol/Vol) oder ein Gemisch aus Dichlormethan und Ethanol im Verhältnis 9:1 (Vol/Vol).

### 2. Stufe

Das nach obenstehender Vorschrift der 1. Stufe erhaltene N-substituierte Indol wird unter Stickstoffatmosphäre in einem aprotischen oder unpolaren organischen Lösungsmittel, wie beispielsweise Diethylether, Methyl-tert.-butylether, Tetrahydrofuran, Dioxan, Toluol, Xylol, Methylenchlorid oder Chloroform gelöst und zu einer unter Stickstoff-Atmosphäre bereiteten Lösung einer einfach molaren bis zu 60-prozentig überschüssigen Menge Oxalylchlorid in einem aprotischen oder unpolaren Lösungsmittel, wie z.B. in Diethylether, Methyl-tert.-butylether, Tetrahydrofuran, Dioxan, Toluol, Xylol, Methylenchlorid gegeben, wobei die Temperatur zwischen -5°C und 20°C gehalten wird. Man erhitzt sodann die Reaktionslösung bei einer Temperatur zwischen 10°C und 130°C, vorzugsweise zwischen 20°C und 80°C, besonders zwischen 30°C und 50°C für einen Zeitraum von 30 Minuten bis zu 5 Stunden und dampft anschließend das Lösungsmittel ab. Der verbleibende Rückstand des auf diese Weise gebildeten "Indolyl-3-glyoxylsäurechlorids" wird in einem aprotischen Lösungsmittel wie z.B. Tetrahydrofuran, Dioxan, Diethylether, Toluol oder auch in einem dipolar aprotischen Lösungsmittel, wie z.B. Dimethylformamid, Dimethylacetamid oder Dimethylsulfoxid gelöst, auf eine Temperatur zwischen 10°C und -15°C, vorzugsweise zwischen -5°C und 0°C, gekühlt und in Gegenwart eines Säurefängers mit einer Lösung des primären oder sekundären Amins in einem Verdünnungsmittel versetzt. Als Verdünnungsmittel kommen die oben zur Auflösung des Indolyl-3-glyoxylsäurechlorids verwendeten Lösungsmittel in Frage. Als Säurefänger finden Triethylamin, Pyridin, Dimethylaminopyridin, bas. Ionenaustauscher. Natriumcarbonat, Kaliumcarbonat, pulverisiertes Kaliumhydroxid sowie überschüssiges, zur Reaktion eingesetztes, primäres oder sekundäres Amin Verwendung. Die Reaktion findet bei einer Temperatur von 0°C bis 120°C, vorzugsweise bei 20°-80°C, besonders zwischen 40°C und 60°C statt. Nach 1-3 stündiger Reaktionszeit und 24-stündigem Stehen bei Raumtemperatur wird das Hydrochlorid des Säurefängers filtriert, das Filtrat i. Vak. eingeengt und der Rückstand aus einem organischen Lösungsmittel umkristallisiert oder durch Säulenchromatographie über Kieselgel oder Aluminiumoxid gereinigt. Als Laufmittel findet z.B. ein Gemisch aus Dichlormethan und Ethanol (95:5, Vol/Vol) Verwendung.

### 3. Stufe

Das nach obenstehender Vorschrift (2. Stufe) erhaltene N-Nitrobenzyl-substituierte "Indolglyoxylsäureamid" wird in einem protischen Lösungsmittel, wie z.B. Methanol, Ethanol, Propanol, Isopropanol bzw. Butanol oder in einem unpolaren Lösungsmittel, wie z.B. Tetrahydrofuran, Dioxan oder Glykoldimethylether oder in einem dipolar aprotischen Lösungsmittel, wie z.B. Dimethylsulfoxyd, Dimethylformamid, Dimethylacetamid bzw. N-Methylpyrrolidon gelöst und die Lösung unter Stickstoff-Atmosphäre und Rühren mit einem Hydrierkatalysator wie z.B. Raney-Nickel, Palladium/Kohle oder Platin versetzt. In die Suspension wird Wasserstoff unter mäßigem Schütteln bei einem Gasdruck von 1-15 bar, vorzugsweise 2-10 bar, besonders bei 4-6 bar eingeleitet und die Temperatur auf ca. 20°-80°C, vorzugsweise 30°-60°C, besonders auf 45°-55°C erhöht. Gegebenenfalls wird nach ca. 1 Stunde nochmals eine Katatysatormenge zugegeben und die Hydrierung fortgesetzt. Nach einer Reaktionszeit von 4-10 Stunden war die Hydrierung beendet. Man filtriert den Katalysator unter Stickstoffatmosphäre, engt das Lösungsmittel im Vakuum zur Trockne ein und trocknet den farblosen bis gelblichen Rückstand i. Vak. bei 40°C.

### Ausführungsbeispiele

Gemäß dieser allgemeinen Vorschrift für die Stufen 1 -3, denen das Syntheseschema 1 zugrundeliegt, wurden folgende Verbindungen synthetisiert, die unter Angabe der jeweiligen chemischen Bezeichnung aus der nachfolgenden Übersicht hervorgehen:

### Beispiel 1

### N-(Pyridin-4-yl)-[1-(4-aminobenzyl)-indol-3-yl] glyoxylsäureamid (D-68838)

### 1.Stufe

### 1-(4-Nitrobenzyl)-indol

Eine Mischung von 5,28 g Natriumhydrid (0,22 Mol, Mineralölsuspension) in 200 ml Dimethyl-sulfoxid wird mit einer Lösung von 23,4 g (0,2 Mol) Indol in 100 ml Dimethylsulfoxid versetzt. Man erhitzt 1 Stunde auf 65°C, läßt danach abkühlen und tropft sodann 37,7 g (0,22 Mol) 4-Nitrobenzyl-chlorid zu. Die Lösung wird auf 60°C erwärmt, 14 Stunden bei Raumtemperatur aufbewahrt und sodann unter Rühren in 700 ml Wasser gegossen. Man extrahiert portionsweise mit insgesamt 300 ml Methylenchlorid, trocknet die organische Phase mit wasserfreiem Natriumsulfat, filtriert und engt das Filtrat im Vakuum ein. Der Rückstand wird über eine Kieselgelsäule gereinigt (Kieselgel 60. Fa. Merck AG, Dannstadt; Laufmittel Methylenchlorid/Ethanol 9:1, V/V).

### Ausbeute: 43,9 g (87% d.Th.)

MS: m/e 253 (M+H)

### 2. Stufe

### N-(Pyridin-4-yl)-[1-(4-nitrobenzyl)-indol-3-yl]glyoxylsäureamid (D-68836)

Eine Lösung von 4,50 ml Oxalylchlorid in 50 ml Ether wird bei O°C und unter Stickstoffatmosphäre tropfenweise mit einer Lösung von 10,09 g (0,04 Mol) 1-(4-Nitrobenzyl)-indol in 50 ml Ether versetzt. Es wird 2 Stunden bei Rückflußtemperatur erhitzt und anschließend das Lösungsmittel abgedampft. Zum Rückstand gibt man 100 ml Tetrahydrofuran, kühlt auf -5°C und gibt tropfenweise eine Lösung von von 9,32 g (0,099 Mol) 4-Aminopyridin in 400 ml Tetrahydrofuran zu. Man erhitzt 3 Stunden zum Rückfluß und läßt über Nacht bei Raumtemperatur stehen. Das 4-Aminopyridin Hydrochlorid wird abgesaugt, der Niederschlag mit Tetrahydrofuran gewaschen, das Filtrat i. Vak. eingeengt und der Rückstand aus Essigester umkristallisiert.

### Ausbeute: 13,5 g (84% d. Th.)

MS: m/e 401 (M+H)

### 3.Stufe

### N-(Pyridin-4-yl)-[1-(4-aminobenzyl)-indol-3-yl]glyoxylsäureamid (D-68838)

Eine Mischung aus 200 mg Raney Nickel in 50 ml Dioxan wird mit einer Suspension von 320 mg (0,8 mMol) N-(Pyridin-4-yl)-[1-(4-nitrobenzyl)-indol-3-yl]-glyoxylsäureamid in einem Lösungsmittelgemisch von 150 ml Dioxan und 20 ml Isopropanol versetzt. In diese Suspension wird unter Schütteln Wasserstoff bei einem Gasdruck von 5 bar eingeleitet und die Temperatur bei 30-35°C gehalten. Nach ca. 3 Stunden werden nochmals 400 mg Raney Nickel zugesetzt und die Hydrierung unter lebhaftem Schütteln nochmals 8 Stunden bei 35°C und 5 bar fortgesetzt. Man filtriert den Katalysator unter N₂-Atmosphäre, engt das Filtrat im Vakuum zur Trockne ein und trocknet den Rückstand im Vakuum bei 40°C.

### Ausbeute: 273 mg (92% d.Th.)

MS: m/e 371 (M+H)

Ferner lassen sich die Verbindungen der allgemeinen Formel 1 mit Z=O, R=NO₂ und NH₂ und R₂= Aryl, Aralkyl, Heteroaryl, Heteroaralkyl und der Allylaminocarbonyl-2-methyl-prop-1-yl-Gruppe auch nach dem Syntheseweg des Schemas 2 synthetisieren:

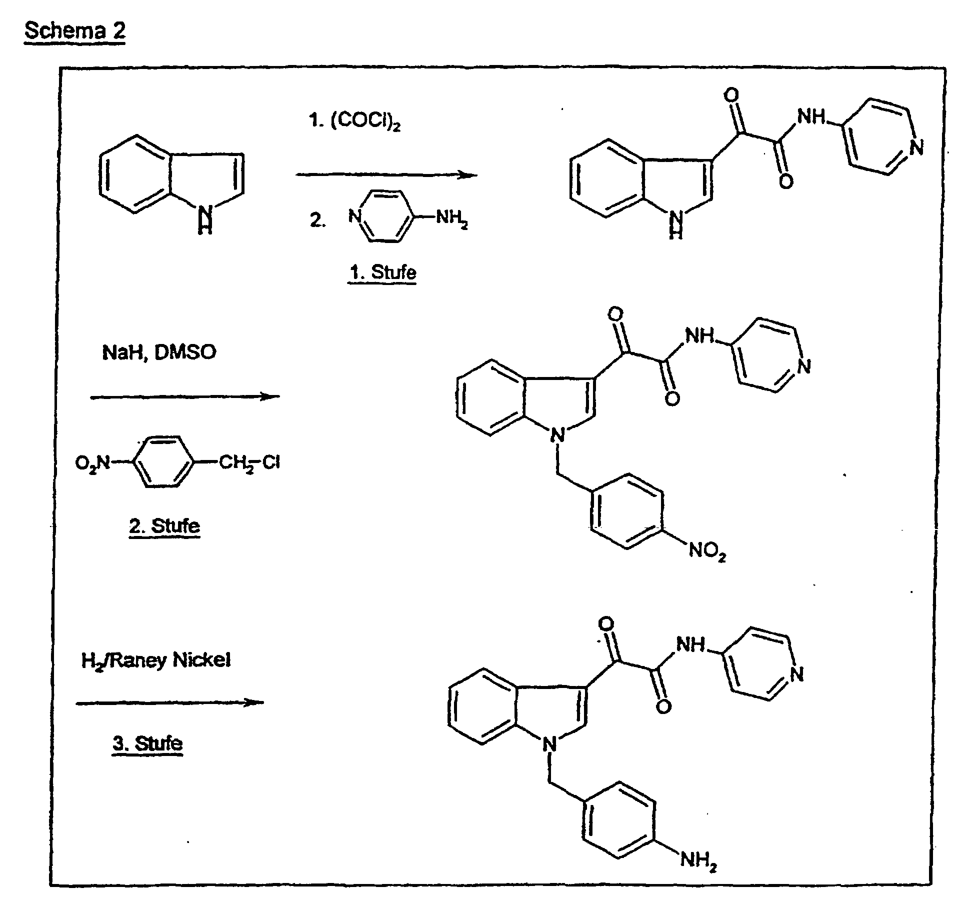

### N-(Pyridin-4-yl)-[1-(4-aminobenzyl)indol-3-yl]glyoxylsäureamid

### 1. Stufe

### N-(Pyridin-4-yl)-(indol-3-yl)glyoxylsäureamid

Zu einer Lösung von 9 ml Oxalylchlorid in 100 ml wasserfreiem Ether wird tropfenweise bei 0°C eine Lösung von 10 g (85,3 mMol) Indol in 100 ml Ether zugegeben. Man hält das Gemisch 3 Stunden unter Rückfluß. Sodann wird bei -5°C eine Suspension von 12 g (127,9 mMol) 4-Amino-pyridin in 500 ml Tetrahydrofuran zugetropft, das Reaktionsgemisch unter Rühren 3 Stunden auf Rückflußtemperatur erhitzt und über Nacht bei Raumtemperatur stehengelassen. Man filtrierte, behandelte den Niederschlag mit Wasser und reinigte die getrocknete Verbindung über eine Kieselgelsäule (Kieselgel 60, Fa. Merck AG, Darmstadt) unter Anwendung des Elutionsmittels Methylenchlorid/Ethanol (10:1, v/v).

### Ausbeute: 9,8 g (43,3% d.Th.)

MS: m/e 266 (M+H)

### 2. Stufe

### N-(Pyridin-4-yl)-[1-(4-nitrobenzyl)-indol-3-yl] glyoxylsäureamid (D-68836)

Das nach der 1. Stufe (Schema 2) erhaltene N-(Pyridin-4-yl)-indol-3-yl))glyoxylsäureamid wird gemäß der "Benzylierungsvorschrift" (Seite 5) mit 4-Nitrobenzylchlorid umgesetzt und die erhaltene Verbindung N-(Pyridin-4-yl)-[1-(4-nitrobenzyl) indol-3-yl] glyoxylsäureamid isoliert.

Ausbeute: 64% d.Th.

MS: m/e 401 (M+H)

### 3. Stufe

### N-(Pyridin-4-yl)-[1-(4-aminobenzyl)indol-3-yl]glyoxylsäureamid (D-68838)

Das nach der 2. Stufe (Schema 2) erhaltene N-(Pyridin-4-yl)-[1-(4-nitrobenzyl)-indol-3-yl] glyoxylsäureamid wird gemäß der "Hydriervorschrift (Seite 7) Katalytisch hydriert und die erhaltene Verbindung N-(Pyridin-4-yl)-[1-(4-aminobenzyl) indol-3-yl] glyoxylsäureamid isoliert.

Ausbeute: 94% d.Th.

### MS: m/e 371 (M+H)

### Allgemeine Vorschrift zur Darstellung der Verbindungen der allgemeinen Formel 1 gemäß Schema 2

### 1. Stufe:

Zu einer unter Stickstoffatmophäre bereiteten Lösung einer einfach molaren bis zu 60% überschüssigen Menge Oxalylchlorid in einem aprotischen oder unpolaren Lösungsmittel, wie z.B. in Diethylether, Methyl-tert.-butylether, Tetrahydrofuran, Dioxan oder auch Dichlormethan, wird bei einer Temperatur zwischen-5°C und +5°C tropfenweise das in einem Lösungsmittel, wie z.B. oben für Oxalylchlorid angegeben, gelöste Indol-Derivat, das unsubstituiert oder an C-2 bzw. im Phenylring substituiert sein kann, zugegeben. Man erhitzt sodann die Reaktionslösung für 1 bis zu 5 Stunden auf eine Temperatur zwischen 10°C und 120°C, vorzugsweise zwischen 20°C und 80°C, besonders zwischen 30°C und 60°C und dampft anschließend das Lösungsmittel ab. Der verbleibende Rückstand des (Indol-3-yl) glyoxylsäurechlorids wird in einem aprotischen Lösungsmittel, wie z.B. Tetrahydrofuran, Dioxan, Diethylether, Toluol oder auch in einem dipolar aprotischen ' Lösungsmittel, wie z.B. Dimethylformamid, Dimethylacetamid oder Dimethylsulfoxid gelöst bzw. suspendiert, auf eine Temperatur zwischen -10°C und +10°C, vorzugsweise auf -5°C bis 8°C gekühlt und in Gegenwart eines Säurefängers mit einer Lösung des primären oder sekundären Amins in einem Verdünnungsmittel versetzt. Als Verdünnungsmittel kommen die zur Auflösung des "Indolyl-3-glyoxylsäurechlorids" verwendeten Lösungsmittel in Frage. Als Säurefänger finden Triethylamin, Pyridin, Dimethylaminopyridin, bas. Ionenaustauscher, Natriumcarbonat, Kaliumcarbonat, pulverisiertes Kaliumhydroxid sowie überschüssiges, zur Reaktion eingesetztes primäres oder sekundäres Amin Verwendung. Die Reaktion findet bei einer Temperatur von 0°C bis 120 °C, vorzugsweise bei 20-80°C, besonders zwischen 40°C und 60°C statt. Nach 1-4-stündiger Reaktionszeit und 24-stündigem Stehen bei Raumtemperatur wird filtriert, der Niederschlag mit Wasser digeriert, abgesaugt und im Vakuum getrocknet. Man reinigt die gewünschte Verbindung durch Umkristallisation in einem organischen Lösungsmittel oder durch Säulenchromatographie an Kieselgel oder Aluminiumoxid. Als Laufmittel findet z.B. ein Gemisch aus Dichlormethan und Ethanol (10:1, vol/vol) Verwendung.

### 2. Stufe

Das nach obenstehender Vorschrift der 1. Stufe erhaltene "Indol-3-yl-glyoxylsäureamid" wird in einem protischen, dipolar aprotischen oder unpolaren organischen Lösungsmittel, wie z.B. in Isopropanol, Tetrahydrofuran, Dimethylsulfoxid, Dimethylformamid, Dimethylacetamid, N-Methylpyrrolidon, Dioxan, Toluol oder Methylenchlorid gelöst und tropfenweise zu einer in einem Dreihalskolben unter N₂-Atmosphäre vorbereiteten molaren oder überschüssig eingesetzten Suspension einer Base, wie z.B. Natriumhydrid, pulverisiertes Kaliumhydroxid, Kalium-tert.-butylat, Dimethylaminopyridin oder Natriumamid, in einem geeigneten Lösungsmittel gegeben. Sodann gibt man das gewünschte Alkyl-, Aralkyl-bzw. Heteroaralkylhalogenid entweder unverdünnt oder in einem Verdünnungsmittel, das z.B. auch zur Lösung des "Indol-3-yl-glyoxylsäureamids" verwendet wurde, gegebenenfalls unter Zusatz eines Katalysators, wie z.B. Kupfer, zu und läßt einige Zeit, z.B. 30 Minuten bis 12 Stunden, reagieren und hält die Temperatur innerhalb eines Bereichs zwischen 0°C und 120°C, vorzugsweise zwischen 30°C und 80°C, besonders zwischen 50°C und 70°C. Nach Beendigung der Reaktion wird das Reaktionsgemisch in Wasser gegeben, die Lösung z.B. mit Diethylether, Dichlormethan, Chloroform, Methyl-tert.-butylether, Tetrahydrofuran bzw. n-Butanol extrahiert und die jeweils erhaltene organische Phase mit wasserfreiem Natriumsulfat getrocknet. Man engt die organische Phase im Vakuum ein, kristallisiert den verbleibenden Rückstand durch Anreiben bzw. reinigt den öligen Rückstand durch Destillation oder durch Säulen- bzw. Flashchromatographie an Kieselgel oder Aluminiumoxid. Als Laufmittel dient beispielsweise ein Gemisch aus Methylenchlorid und Diethylether im Verhältnis 8:2 (Vol/Vol) oder ein Gemisch aus Methylenchlorid und Ethanol im Verhältnis 9:1 (V/V).

### 3. Stufe

Das nach obenstehender Vorschrift (2.Stufe) erhaltene N-Nitrobenzyl-substituierte "Indolglyoxylsäureamid" wird in einem protischen Lösungsmittel, wie z.B. Methanol, Ethanol, Propanol bzw. Butanol oder in einem unpolaren Lösungsmittel, wie z.B. Tetrahydrofuran, Dioxan odr Glykoldimethylether oder in einem dipolar aprotischen Lösungsmittel, wie z.B. Dimethylsulfoxid, Dimethylformamid, Dimethylacetamid bzw. N-Methylpyrrolidon gelöst und die Lösung unter Stickstoff-Atmosphäre und Rühren mit einem Hydrierkatalysator wie z.B. Raney-Nickel, Palladium/Kohle oder Platin versetzt. In die Suspension wird Wasserstoff unter mäßigem Schütteln bei einem Gasdruck von 1-15 bar, vorzugsweise 2-10 bar, besonders bei 4-6 bar eingeleitet und die Temperatur auf ca. 20°-80°C, vorzugsweise 30°-60°C, besonders auf 45°-55°C erhöht. Gegebenenfalls wird nach ca. 1 Stunde nochmals eine Katalysatormenge zugegeben und die Hydrierung fortgesetzt. Nach einer Reaktionszeit von 4-6 Stunden war die Hydrierung beendet. Man filtriert den Katalysator unter Stickstoffatmosphäre, engt das Lösungsmittel zur Trockne ein und trocknet den farblosen bis gelblichen Rückstand im Vakuum bei 40°C.

Gemäß dieser allgemeinen Vorschrift für die Stufen 1-3, denen das Syntheseschema 2 zugrundeliegt, wurden die Verbindungen D-68836 und D-68838 synthetisiert, die auch schon gemäß des Syntheseablaufs des Reaktionsschemas 1 dargestellt wurden.

In einem Tubulin-Polymerisations-Assay konnte die Aktivität der vorliegenden Verbindungen gezeigt werden. Insbesondere wurde unter Beweis gestellt, daß D-68838 die Polymerisation von Tubulin hemmt und somit einen destabilisierenden Effekt auf Mikrotubuli bzw. die mitotische Spindel ausübt.

## Patentansprüche

1. Neue, substituierte N-Benzyl-Indol-3-yl- glyoxylsäure-Derivate mit Antitumorwirkung der allgemeinen Formel 1 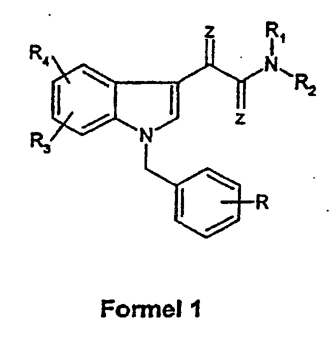 Wobei die Reste R, R₁, R₂, R₃, R₄ und Z die folgende Bedeutung haben:
R = Nitro, Amino, Mono- bzw. Di(C₁-C₆)-alkylamino, Mono- bzw. Di(C₁-C₆)-cycloalkylamino, (C₁-C₆)-Acylamino, Phenyl (C₁-C₆)-alkylamino, Aroylamino, Heteroaroylamino, (C₁-C₆)-Alkylsulfonamido, Arylsulfonamido, Maleinimido, Succinimido, Phthalimido, Benzyloxycarbonylamino (Z-Amino), tert.-Butoxycarbonylamino (BOC-Amino), 9-Fluorenylmethoxycarbonylamino (Fmoc-Amino), Triphenylmethylamino (Tr-Amino), 2-(4'-Pyridyl)-ethoxycarbonylamino (Pyoc-Amino), Diphenylmethylsilyl-amino (DPMS-Amino), wobei die Reste für R wahlweise an den C-Atomen 2,3 und 4 des Phenylrings substituiert sein können,
R kann weiterhin für den Fall, daß R₁=Wasserstoff, die Methyl-oder Phenylmethylgruppe sowie den Benzyloxycarbonyl-Rest (Z-Rest), den tert.-Butoxycarbonyl-Rest (BOC-Rest) und die Acetylgruppe darstellt, die folgenden Reste bedeuten:
- NH-CH₂-COOH; -NH-CH(CH₃)-COOH;
(CH₃)₂CH-CH₂-CH₂-CH(NH)-COOH; H₃C-CH₂-CH(CH₃)-CH(NH)-COOH;
HOH₂C-CH(NH)-COOH; Phenyl-CH₂-CH(NH)-COOH; (4-Imidazoyl)-CH₂-CH(NH)-COOH;
HN=C(NH₂)-NH-(CH₂)₃-CH(NH)-COOH; H₂N-(CH₂)₄-CH(NH)-COOH;
H₂N-CO-CH₂-CH(NH)-COOH; HOOC-(CH₂)₂-CH(NH)-COOH
R₁ = Wasserstoff, (C₁-C₆)-Alkyl, wobei die Alkylgruppe ein- oder mehrfach durch den Phenylring substituiert sein kann und dieser Phenylring seinerseits ein- oder mehrfach durch Halogen, (C₁-C₆)-Alkyl, (C₃-C₇)-Cycloalkyl, durch Carboxylgruppen, mit C₁-C₆-Alkanolen veresterte Carboxylgruppen, Trifluormethylgruppen, Hydroxylgruppen, Methoxygruppen, Ethoxygruppen, Benzyloxygruppen sowie durch eine im Phenylteil ein- oder mehrfach mit (C₁-C₆)- Alkylgruppen, Halogenatomen oder Trifluormethylgruppen substituierte Benzylgruppe substituiert sein kann,
R₁ steht ferner für die Benzyloxycarbonyl-Gruppe (Z-Gruppe) und für den tertiär-Butoxycarbonylrest (Boc-Rest), weiterhin für die Acetylgruppe
R₂ kann den Phenylring, der ein- oder mehrfach mit (C₁-C₆)-Alkyl, (C₁-C₆)-Alkoxy, Cyano, Halogen, Trifluormethyl, Hydroxy, Benzyloxy, Nitro, Amino, (C₁-C₆)-Alkylamino, (C₁-C₆)-Alkoxycarbonyl-amino und mit der Carboxylgruppe bzw. mit der mit C₁-C₆- Alkanolen veresterten Carboxylgruppe substituiert ist, oder ein Pyridin-Gerüst der Formel 2 und deren N-Oxid 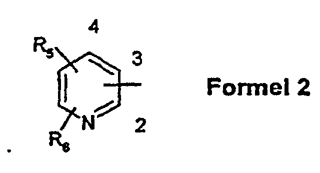 bedeuten und dessen N-Oxid, wobei das Pyridin-Gerüst wahlweise an den Ringkohlenstoff Atomen 2, 3 und 4 gebunden ist und mit den Substituenten R₅ und R₆ substituiert sein kann
R₅ und R₆ in der Formel 2 können gleich oder verschieden sein und die Bedeutung (C₁-C₆)-Alkyl sowie die Bedeutung (C₃-C₇)-Cycloalkyl, (C₁-C₆)-Alkoxy, Nitro, Amino, Hydroxy, Halogen und Trifluormethyl besitzen und ferner den Ethoxycarbonylamino-Rest sowie die Gruppe Carboxyalkyloxy darstellen, bei dem die Alkylgruppe über 1-4 C-Atome verfügen kann.
R₂ kann ferner ein 2-bzw. 4-Pyrimidinyl-Heterocyclus sein, wobei der 2-Pyrimidinyl-Ring ein- oder mehrfach mit der Methylgruppe substituiert sein kann, weiterhin das mit (C₁-C₆)-Alkyl, Halogen, der Nitrogruppe, der Aminogruppe und dem (C₁-C₆)-Alkylamino-Rest substituierte 2-,3-,4-,5-,6-,7- und 8-Chinolylgerüst bedeuten, eine 2-, 3- und 4-Chinolylmethylgruppe darstellen, wobei die Ringkohlenstoffe des Pyridylmethylrestes der Chinolylgruppe und des Chinolylmethyl-Restes mit (C₁-C₆)-Alkyl, (C₁-C₆)-Alkoxy, Nitro, Amino und (C₁-C₆)-Alkoxycarbonylamino substituiert sein können
R₂ kann weiterhin für den Fall, daß R₁=Wasserstoff, die Methyl-oder Benzylgruppe sowie den Benzyloxycarbonyl-Rest (Z-Rest), den tert.-Butoxycarbonyt-Rest (BOC-Rest) und die Acetylgruppe darstellt, die folgenden Reste bedeuten:
-CH₂COOH;-CH(CH₃)-COOH;(CH₃)₂CH-(CH₂)₂-CH(COOH)-; H₃C-H₂C-CH(CH₃)-CH(COOH)-;
HO-H₂C-CH(COOH)-; Phenyl-CH₂-CH(COOH)-; (4-Imidazolyl)-CH₂-CH(COOH)-;
HN=C(NH₂)-NH-(CH₂)₃-CH(COOH)-; H₂N-(CH₂)₄-CH(COOH)-; H₂N-CO-CH₂-CH(COOH)-;
HOOC-(CH₂)₂-CH(COOH)-;
R₂ kann weiterhin für den Fall, daß R₁ Wasserstoff, dieZ-Gruppe, den BOC-Rest, die Acetyl- oder die Benzylgruppe bedeuten, der Säurerest einer natürtichen oder unnatürlichen Aminosäure sein, z.B. den α-Glycyl-, den α-Sarkosyl-, den α-Alanyl-, den α-Leucyl-, den α-iso-Leucyl-, den α-Seryl-, den α-Phenylalanyl-, den α-Histidyl-, den α-Prolyl-, den α-Arginyl-, den α-Lysyl-, den α-Asparagyl- und den α-Glutamyl-Rest darstellen, wobei die Aminogruppen der jeweiligen Aminosäuren ungeschützt vorliegen oder geschützt sein können. Als Schutzgruppe der Aminofunktion kommen der Carbobenzoxy-Rest (Z-Rest) und der tert.-Butoxycarbonyl-Rest (BOC-Rest) sowie die Acetylgruppe in Frage. Im Fall des für R₂ beanspruchten Asparagyl-und Glutamylrestes liegt die zweite, nicht gebundene Carboxylgruppe als freie Carboxylgruppe oder in Form einer mit C₁-C₆-Alkanolen veresterten Carboxylgruppe, z.B. als Methyl-, Ethyl- bzw. als tert.- Butylester vor, weiterhin kann R₂ die Allylaminocarbonyl-2-methyl-prop-1-yl-Gruppe bedeuten;
R₁ und R₂ können ferner zusammen mit dem Stickstoff-Atom, an das sie gebunden sind, einen Piperazinring der Formel 3 oder einen Homopiperazinring bilden, sofern R₂ eine Aminoalkylengruppe darstellt, bei dem 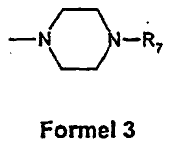 R₇ einen Alkylrest darstellt, einen Phenylring bedeutet, der ein- oder mehrfach mit (C₁-C₆)-Alkyl, (C₁-C₆)-Alkoxy, Halogen, der Nitrogruppe, der Aminofunktion und mit der (C₁-C₆)-Alkylaminogruppe substituiert sein kann; R₇ bedeutet ferner die Benzhydryl-Gruppe und die Bis-p-fluorbenzhydryl-Gruppe.
R₃ und R₄ können gleich oder verschieden sein und Wasserstoff, (C₁-C₆)-Alkyl, (C₃-C₇)-Cycloalkyl, (C₁-C₆)-Alkanoyl, Halogen und Benzyloxy bedeuten; weiterhin können R₃ und R₄ die Nitrogruppe, die Aminogruppe, die (C₁-C₄)-mono- oder dialkylsubstituierte Aminogruppe und die (C₁-C₆)-Alkoxy-carbonylamino-Funktion oder die (C₁-C₆)-Alkoxycarbonylamino-(C₁-C₆)-alkyl-Funktion bedeuten
Z steht für O und S.

2. N-(Pyridin-4-yl)-[1-(4-aminobenzyl)-indol-3-yl] glyoxylsäureamid (D-68838)

3. N-(Pyridin-4-yl)-[1-(4-nitrobenzyl)-indol-3-yl] glyoxylsäureamid (D-68836)

4. Säureadditionssalze der Verbindungen nach Anspruch 1-4 der allgemeinen Formel 1 als Salze von Mineralsäuren, wie Salzsäure, Schwefelsäure, Phosphorsäure, Salze von organischen Säuren, insbesondere Essigsäure, Milchsäure, Malonsäure, Maleinsäure, Fumarsäure, Gluconsäure, Glucuronsäure, Zitronensäure, Ascorbinsäure. Embonsäure, Methansulfonsäure, Trifluoressigsäure, Bemsteinsäure, 2-Hydroxy-ethansulfonsäure, Nicotinsäure und p-Toluol-sulfonsäure.

5. Pharmazeutische Zubereitungen der Verbindungen nach Anspruch 1-4 der allgemeinen Formel 1 mit einem Gehalt an mindestens einer der Verbindungen der Formel 1 oder deren Salze nach Anspruch 3 mit physiologisch verträglichen anorganischen oder organischen Säuren und gegebenenfalls pharmazeutisch verwendbaren Träger- und/oder Verdünnungs- bzw. Hilfsstoffen.

6. Applikationsformen der Verbindungen nach Anspruch 1-4 der allgemeinen Formel 1 mit einem Gehalt an mindestens einer der Verbindungen der Formel 1 oder deren Salze nach Anspruch 3 in Form von Tabletten, Dragees, Kapseln, Lösungen zur Infusion oder Ampullen, Suppositorien, Pflaster, inhalativ einsetzbare Pulverzubereitungen, Suspensionen, Cremes und Salben

7. Verwendung der Verbindungen nach Anspruch 1-4 der allgemeinen Formel 1 zur Herstellung von Antitumormitteln.

8. Verwendung von Verbindungen nach Anspruch 1-4 der allgemeinen Formel 1 zur Behandlung von Tumorerkrankungen.

## Claims

1. Novel, substituted N-benzylindol-3-ylglyoxylic acid derivatives having antitumour action
of the general formula 1 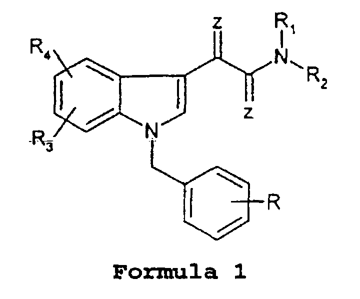 Where the radicals R, R₁, R₂, R₃, R₄ and Z have the following meaning:
R = nitro, amino, mono- or di(C₁-C₆)-alkylamino, mono- or di(C₁-C₆)-cycloalkylamino, (C₁-C₆)-acylamino, phenyl (C₁-C₆)-alkylamino, aroylamino, heteroaroylamino, (C₁-C₆)-alkylsulphonamido, arylsulphonamido, maleimido, succinimido, phthalimido, benzyloxycarbonylamino (Z-amino), tert-butoxycarbonylamino (BOC-amino), 9-fluorenylmethoxycarbonylamino (Fmoc-amino), triphenylmethylamino (Tr-amino), 2-(4'-pyridyl)ethoxycarbonylamino (Pyoc-amino), diphenylmethylsilylamino (DPMS-amino), where the radicals for R can be selectively attached to the C atoms 2, 3 and 4 of the phenyl ring,
R can furthermore be, in the case in which R₁=hydrogen, the methyl or phenylmethyl group and the benzyloxycarbonyl radical (Z radical), the tert-butoxycarbonyl radical (BOC radical) and the acetyl group, the following radicals:
- NH-CH₂-COOH; -NH-CH(CH₃)-COOH;
(CH₃)₂CH-CH₂-CH₂-CH(NH) -COOH; H₃C-CH₂-CH(CH₃)-CH(NH)-COOH;
HOH₂C-CH(NH)-COOH; phenyl-CH₂-CH(NH)-COOH;
(4-imidazoyl)-CH₂-CH(NH)-COOH;
HN=C(NH₂)-NH-(CH₂)₃-CH(NH)-COOH; H₂N-(CH₂)₄-CH(NH)-COOH;
H₂N-CO-CH₂-CH(NH)-COOH; HOOC- (CH₂)₂-CH(NH)-COOH
R₁ = hydrogen, (C₁-C₆)-alkyl, where the alkyl group can be mono- or polysubstituted by the phenyl ring and this phenyl ring for its part can be mono- or polysubstituted by halogen, (C₁-C₆)-alkyl, (C₃-C₇)-cycloalkyl, by carboxyl groups, carboxyl groups esterified with C₁-C₆-alkanols, trifluoromethyl groups, hydroxyl groups, methoxy groups, ethoxy groups, benzyloxy groups and by a benzyl group which is mono- or polysubstituted in the phenyl moiety by (C₁-C₆)-alkyl groups, halogen atoms or trifluoromethyl groups,
R₁ is further the benzyloxycarbonyl group (Z group) and the tertiary-butoxycarbonyl radical (Boc radical), furthermore the acetyl group,
R₂ can be the phenyl ring, which is mono- or polysubstituted by (C₁-C₆) -alkyl, (C₁-C₆)-alkoxy, cyano, halogen, trifluoromethyl, hydroxyl, benzyloxy, nitro, amino, (C₁-C₆)-alkylamino, (C₁-C₆)-alkoxycarbonylamino and by the carboxyl group or by the carboxyl group esterified with C₁-C₆-alkanols, or can be a pyridine structure of the formula 2 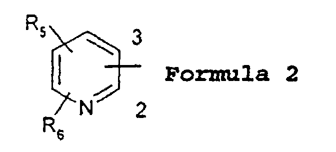 and its N-oxide, where the pyridine structure is selectively attached at the ring carbon atoms 2, 3 and 4 and can be substituted by the substituents R₅ and R₆,
R₅ and R₆ in formula 2 can be identical or different and have the meaning (C₁-C₆)-alkyl and the meaning (C₃-C₇)-cycloalkyl, (C₁-C₆)-alkoxy, nitro, amino, hydroxyl, halogen and trifluoromethyl and further are the ethoxycarbonylamino radical and the group carboxyalkyloxy in which the alkyl group can have 1-4 C atoms,
R₂ can further be a 2- or 4-pyrimidinyl heterocycle, where the 2-pyrimidinyl ring can be mono- or polysubstituted by the methyl group, furthermore can be the 2-, 3-, 4-, 5-, 6-, 7- and 8-quinolyl structure substituted by (C₁-C₆)-alkyl, halogen, the nitro group, the amino group and the (C₁-C₆)-alkylamino radical, can be a 2-, 3- or 4-quinolylmethyl group, where the ring carbons of the pyridylmethyl radical of the quinolyl group and of the quinolylmethyl radical can be substituted by (C₁-C₆)-alkyl, (C₁-C₆)-alkoxy, nitro, amino and (C₁-C₆)-alkoxycarbonylamino,
R₂ in the case in which R₁=hydrogen, the methyl or benzyl group and the benzyloxycarbonyl radical (Z radical), the tert-butoxycarbonyl radical (BOC radical) and the acetyl group can furthermore be the following radicals:
- CH₂COOH; -CH(CH₃)-COOH; (CH₃)₂CH-(CH₂)₂-CH(COOH)-;
H₃C-H₂C-CH(CH₃)-CH(COOH)-; [HO-H₂C-CH(COOH)-;
phenyl-CH₂-CH(COOH)-; (4-imidazolyl)-CH₂-CH(COOH)-;
HN=C(NH₂)-NH-(CH₂)₃-CH(COOH)-; H₂N-(CH₂)₄-CH(COOH)-;
H₂N-CO-CH₂-CH(COOH)-; HOOC-(CH₂)₂-CH(COOH)-;
R₂ in the case in which R₁ is hydrogen, the Z group, the BOC radical, the acetyl or the benzyl group can furthermore be the acid radical of a natural or unnatural amino acid, e.g. the α-glycyl, the α-sarcosyl, the α-alanyl, the α-leucyl, the α-isoleucyl, the α-seryl, the α-phenylalanyl, the α-histidyl, the α-prolyl, the α-arginyl, the α-lysyl, the α-asparagyl and the α-glutamyl radical, where the amino groups of the respective amino acids can be present unprotected or can be protected. A possible protective group of the amino function is the carbobenzoxy radical (Z radical) and the tert-butoxycarbonyl radical (BOC radical) as well as the acetyl group. In the case of the asparagyl and glutamyl radical claimed for R₂, the second, unbonded carboxyl group is present as a free carboxyl group or in the form of a carboxyl group esterified with C₁-C₆-alkanols, e.g. as a methyl, ethyl or as a tert-butyl ester; furthermore, R₂ can be the allylaminocarbonyl-2-methylprop-1-yl group;
R₁ and R₂ can further form, together with the nitrogen atom to which they are bonded, a piperazine ring of the formula 3 or a homopiperazine ring, provided R₂ is an aminoalkylene group, in which 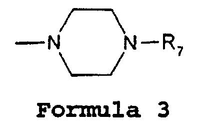
R₇ is an alkyl radical, is a phenyl ring which can be mono- or polysubstituted by (C₁-C₆)-alkyl, (C₁-C₆)-alkoxy, halogen, the nitro group, the amino function and by the (C₁-C₆)-alkylamino group; R₇ is furthermore the benzhydryl group and the bis-p-fluorobenzhydryl group.
R₃ and R₄ can be identical or different and are hydrogen, (C₁-C₆)-alkyl, (C₃-C₇)-cycloalkyl, (C₁-C₆)-alkanoyl, halogen and benzyloxy; R₃ and R₄ can furthermore be the nitro group, the amino group, the (C₁-C₄)-mono- or dialkyl-substituted amino group, and the (C₁-C₆)-alkoxycarbonylamino function or the (C₁-C₆)-alkoxycarbonylamino-(C₁-C₆)-alkyl function,
Z is O and S.

2. N-(Pyridin-4-yl)-[1-(4-aminobenzyl)indol-3-yl]-glyoxylamide (D-68838).

3. N-(Pyridin-4-yl)-[1-(4-nitrobenzyl)indol-3-yl]-glyoxylamide (D-68836).

4. Acid addition salts of the compounds according to Claims 1-4 of the general formula 1 as salts of mineral acids, such as hydrochloric acid, sulphuric acid, phosphoric acid, salts of organic acids, in particular acetic acid, lactic acid, malonic acid, maleic acid, fumaric acid, gluconic acid, glucuronic acid, citric acid, ascorbic acid, embonic acid, methanesulphonic acid, trifluoroacetic acid, succinic acid, 2-hydroxyethanesulphonic acid, nicotinic acid and p-toluenesulphonic acid.

5. Pharmaceutical preparations of the compounds according to Claims 1-4 of the general formula 1 containing at least one of the compounds of the formula 1 or their salts according to Claim 3 with physiologically tolerable inorganic or organic acids and, if appropriate, pharmaceutically utilizable vehicles and/or diluents or excipients.

6. Application forms of the compounds according to Claims 1-4 of the general formula 1 containing at least one of the compounds of the formula 1 or their salts according to Claim 3 in the form of tablets, coated tablets, capsules, solutions for infusion or ampoules, suppositories, patches, powder preparations which can be employed by inhalation, suspensions, creams and ointments.

7. Use of the compounds according to Claims 1-4 of the general formula 1 for the production of antitumour compositions.

8. Use of compounds according to Claims 1-4 of the general formula 1 for the treatment of oncoses.

## Revendications

1. Nouveaux dérivés d'acide N-benzyl-indol-3-yl-glyoxylique substitués à action anti-tumorale de formule générale 1 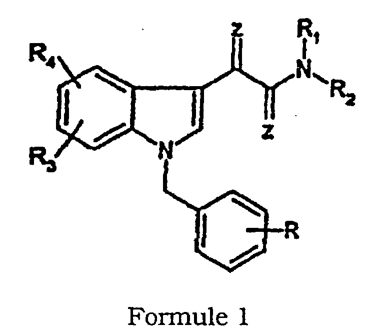 dans laquelle les radicaux R, R₁, R₂, R₃, R₄ et Z ont la signification suivante :
R = nitro, amino, mono- ou selon les cas di-alkyle en C₁ à C₆-amino, mono- ou selon les cas di-cycloalkyle en C₁ à C₆-amino, acylamino en C₁ à C₆, phényl-alkylamino en C₁ à C₆, aroylamino, hétéroaroylamino, alkyle en C₁ à C₆-sulfonamido, arylsulfonamido, maléinimido, succinimido, phtalimido, benzyloxycarbonyl amino (Z-amino), tert.-butoxycarbonylamino (BOC-amino), 9-fluorénylméthoxycarbonyl amino (Fmoc-amino), triphénylméthylamino (Tr-amino), 2-(4'-pyridyl)-éthoxycarbonylamino (Pyoc-amino), diphénylméthyl silyl-amino (DPMS-amino), les restes pour R pouvant au choix être substitués sur les atomes de carbone 2, 3 et 4 du noyau phényle,
R peut en outre, dans le cas où R₁ représente un hydrogène, représenter le groupe méthyle ou le groupe phénylméthyle, ainsi que le radical benzyloxy carbonyle (radical Z), le radical tert.-butoxy carbonyle (radical BOC), et le groupe acétyle, les radicaux ayant les significations suivantes :
-NH-CH₂-COOH ; -NH-CH(CH₃)-COOH ;
(CH₃)₂CH-CH₂-CH₂-CH(NH)-COOH ; H₃C-CH₂-CH(CH₃)-CH(NH)-COOH ;
HOH₂C-CH(NH)-COOH, phényl-CH₂-CH(NH)-COOH ; (4-imidazoyl)-CH₂-CH(NH)-COOH ;
HN=C(NH₂)-NH-(CH₂)₃-CH(NH)-COOH ; H₂N-(CH₂)₄-CH(NH)-COOH ;
H₂N-CO-CH₂-CH(NH)-COOH ;
HOOC-(CH₂)₂-CH(NH)-COOH
R₁ représente un hydrogène, un alkyle en C₁ à C₆, le groupe alkyle pouvant être mono- ou polysubstitué par le noyau phényle et ce noyau phényle pouvant être de son côté mono- ou polysubstitué par un halogène, un alkyle en C₁ à C₆, un cycloalkyle en C₃ à C₇, ou par des groupes carboxyle, par des groupes carboxyle estérifiés par des alcanols en C₁ à C₆, par des groupes trifluorométhyle, des groupes hydroxyle, des groupes méthoxy, des groupes éthoxy, des groupes benzyloxy ainsi qu'être mono- ou polysubstitué par un groupe benzyle mono- ou polysubstitué dans la partie phényle par des groupes alkyle en C₁ à C₆, des atomes d'halogène ou des groupes trifluorométhyle,
R₁ représente en outre un groupe benzyloxy carbonyle (groupe Z) et le radical tert.-butoxy carbonyle (radical Boc) ainsi que le groupe acétyle,
R₂ peut représenter le noyau phényle, qui est mono- ou polysubstitué par un alkyle en C₁ à C₆, un alcoxy en C₁ à C₆, un cyano, un halogène, un trifluorométhyle, un hydroxy, un benzyloxy, un nitro, un amino, un alkylamino en C₁ à C₆, un alcoxy en C₁ à C₆ carbonylamino et estérifié avec le groupe carboxyle ou selon les cas des alcanols en C₁ à C₆, ou une ossature pyridine de formule 2 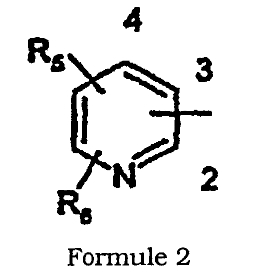 et son N-oxyde, l'ossature de pyridine pouvant au choix être liée aux atomes de carbone cycliques 2, 3 et 4 et pouvant être substituée par les substituants R₅ et R₆, les radicaux R₅ et R₆ peuvent être identiques ou différents et représenter un alkyle en C₁ à C₆ ou un cycloalkyle en C₃ à C₇, un alcoxy en C₁ à C₆, un nitro, un amino, un hydroxy, un halogène ou un trifluorométhyle, et peuvent en outre représenter le radical éthoxycarbonyl amino ainsi que le groupe carboxyalkyloxy dans lequel le groupe alkyle peut disposer 1 à 4 atomes de carbone,
R₂ peut en outre être un hétérocycle 2- ou selon les cas 4-pyrimidinyle, où le noyau 2-pyrimidinyle peut être mono- ou polysubstitué par le groupe méthyle, ainsi que l'ossature 2-, 3-, 4-, 5-, 6-, 7-, et 8-quinoléyle substituée par un alkyle en C₁ à C₆, un halogène, le groupe nitro, le groupe amino et le radical alkylamino en C₁ à C₆, un groupe 2-, 3- et 4-quinoléylméthyle, les atomes de carbone du cycle du radical pyridyl méthyle du groupe quinoléyle et du radical quinoléyl méthyle pouvant être substitués par un alkyle en C₁ à C₆, un alcoxy en C₁ à C₆, un nitro, un amino et un alcoxy en C₁ à C₆-carbonylamino,
R₂ peut en outre, dans le cas où R₁ représente un hydrogène, représenter le groupe méthyle ou le groupe benzyle ainsi que le radical benzyloxy carbonyle (radical Z), le radical tert.-butoxycarbonyle (radical BOC) et le groupe acétyle, et les radicaux ont les significations qui suivent :
-CH₂COOH ; -CH(CH₃)-COOH, (CH₃)₂CH-(CH₂)₂-CH(COOH) ;
H₂C-H₂C-CH(CH₃)-CH(COOH)- ;
HO-H₂C-CH(COOH) ; phényl-CH₂-CH(COOH)- ; (4-imidazolyl)-CH₂-CH(COOH)- ;
HN=C(NH₂)-NH(CH₂)₃-CH(COOH)-; H₂N-(CH₂)₄-CH(COOH)-; H₂N-CO-CH₂-CH(COOH)-; HOOC-(CH₂)₂-CH(COOH)- ;
R₂ peut en outre, dans le cas où R₁ représente un hydrogène, représenter le groupe z, le radical BOC, le groupe acétyle ou le groupe benzyle, le radical acide d'un acide aminé naturel ou non naturel, par exemple le radical α-glycyle, le radical α-sarcosyle, le radical α-alanyle, α-leucyle, α-iso-leucyle, le radical α-séryle, le radical α-phénylalanyle, le radical α-histidyle, le radical α-prolyle, le radical α-arginyle, le radical α-lysyle, le radical α-asparagyle et le radical α-glutamyle, les groupes amino des acides aminés respectifs pouvant se présenter sous forme non protégée ou protégée. Comme groupe protecteur de la fonction amino, il faut mentionner le radical carbobenzoxy (radical Z) et le radical tert.-butoxycarbonyle (radical BOC), ainsi que le groupe acétyle. Dans le cas du radical aspargyle et du radical glutamyle revendiqués pour R₂, le second groupe carboxyle, non lié, se présente sous la forme d'un groupe carboxyle libre ou sous la forme d'un groupe carboxyle estérifié par des alcanols en C₁ à C₆, par exemple sous forme d'ester méthylique, éthylique ou selon les cas tert.-butylique. En outre, R₂ peut représenter le groupe allylamino carbonyl-2-méthyl-prop-1-yle ;
R₁ et R₂ peuvent en outre, ensemble avec l'atome d'azote auquel ils sont liés, former un noyau pipérazine de formule 3 ou un noyau homopipérazine, dans la mesure où R₂ représente un groupe aminoalkylène, dans lequel 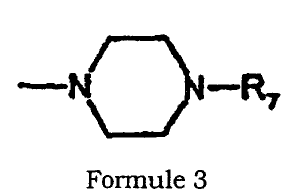
R₇ représente un radical alkyle, un noyau phényle, qui peut être mono-
ou polysubstitué par un alkyle en C₁ à C₆, un alcoxy en C₁ à C₆, un halogène, le groupe nitro, la fonction amino et avec le groupe alkylamino en C₁ à C₆ ; R₇ représente en outre le groupe benzhydryle et le groupe bis-p-fluorobenzhydryle,
R₃ et R₄ peuvent être identiques ou différents et représenter un hydrogène, un alkyle en C₁ à C₆, un cycloalkyle en C₃ à C₇, un alcanoyle en C₁ à C₆, un halogène ou un benzyloxy ; en outre, R₃ et R₄ peuvent représenter le groupe nitro, le groupe amino, le groupe amino mono- ou disubstitué par un alkyle en C₁ à C₄ et la fonction alcoxy en C₁ à C₆-carbonylamino ou la fonction alcoxy en C₁ à C₆-carbonylamino-alkyle en C₁ à C₆,
Z représente O ou S.

2. Amide de l'acide N-(pyridin-4-yl)-[1-(4-aminobenzyl)-indol-3-yl] glyoxylique (D-68838).

3. Amide de l'acide N-(pyridin-4-yl)-[1-(4-nitrobenzyl)-indol-3-yl] glyoxylique (D-68836).

4. Sels d'addition d'acides des composés selon les revendications 1-4 de formule générale 1 sous forme de sels d'acides minéraux, comme de l'acide chlorhydrique, de l'acide sulfurique, de l'acide phosphorique, les sels d'acides organiques, en particulier l'acide acétique, l'acide lactique, l'acide malonique, l'acide maléique, l'acide fumarique, l'acide gluconique, l'acide glucuronique, l'acide citrique, l'acide ascorbique, l'acide embonique, l'acide méthane sulfonique, l'acide trifluoroacétique, l'acide succinique, l'acide 2-hydroxy éthane sulfonique, l'acide nicotinique et l'acide p-toluène sulfonique.

5. Préparations pharmaceutiques des composés selon les revendications 1-4 de formule générale 1, contenant au moins un des composés de formule 1 ou leurs sels selon la revendication 3 avec des acides organiques ou inorganiques physiologiquement acceptables et le cas échéant des supports et/ou diluants ou selon les cas additifs pharmaceutiquement acceptables.

6. Formes d'application des composés selon les revendications 1 à 4, de formule générale 1 contenant au moins un des composés de formule 1 ou leurs sels selon la revendication 3 sous forme de comprimés, de dragées, de gélules, aux fins de transfusion ou d'ampoules, de suppositoires, d'emplâtres, de préparations pulvérulentes utilisables par inhalation, de suspensions, de crèmes et d'onguents.

7. Utilisation des composés selon les revendications 1 à 4, de formule 1 pour la production d'agents anti-tumoraux.

8. Utilisation des composés selon les revendications 1 à 4, de formule 1 pour le traitement des maladies tumorales.
